(19) Europäisches Patentamt
European Patent Office
Office européen des brevets

(11) Veröffentlichungsnummer: **0 132 512**
**A1**

# (12) EUROPÄISCHE PATENTANMELDUNG

(21) Anmeldenummer: 84104671.7

(22) Anmeldetag: 26.04.84

(51) Int. Cl.[4]: **A 01 N 43/70**

(30) Priorität: 24.06.83 DE 3322720

(43) Veröffentlichungstag der Anmeldung: 13.02.85 Patentblatt 85/7

(84) Benannte Vertragsstaaten: AT BE CH DE FR GB IT LI LU NL SE

(71) Anmelder: CHEMISCHE WERKE HÜLS AG
- RSP Patente / PB 15 - Postfach 13 20
D-4370 Marl 1(DE)

(72) Erfinder: Schnurbusch, Horst, Dr.
Overwegstrasse 36
D-4690 Herne 1(DE)

(72) Erfinder: Baltruschat, Helmut, Dr.
Am Hagenbach 3
D-4405 Nottuln(DE)

## (54) Verwendung von in 2-Stellung mit (substituierten) Aminogruppen substituierten 4-DL-Alkylester-alpha-alaninyl-6-chlor-s-Triazinen als Herbizide, insbesondere gegen Flughafer.

(57) Die Erfindung betrifft die Verwendung von in 2-Stellung mit einer (substituierten) Aminogruppe substituierten 4-DL-Alkyester-α- alaninyl-6-chlor-s-triazinen der allgemeinen Formel

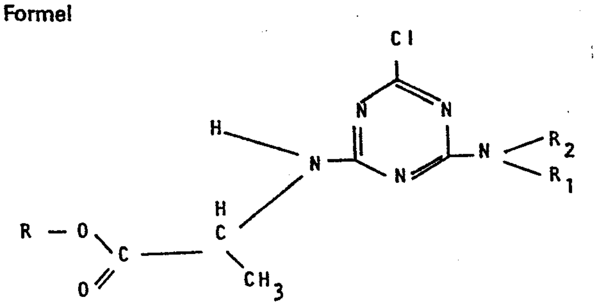

in der $R_1$ einen gerad- oder verzweigtkettigen Alkyl-, Alkoxy-, Alkoxyalkyl oder Alkylol-Rest oder einen gegebenenfalls mit bis zu 5 Methylgruppen substituierter Cyclopentyl- oder Crclohexylring, $R_2$ einen Rest aus der Gruppe der Alkyle mit eine bis drei C-Atomen oder vorzugsweise eine H-Atom und R einen Rest aus der Gruppe der Alkyle mit ein bis drei C-Atomen bedeuten, in geeigneter Applikationsform zur selektiven Behandlung von Nutzpflanzenkulturen mit breitem Wirkungsspektrum bei Unkräutern und Schadgräsern vor und/oder nach dem Auflaufen der Saat.

Insbesondere geeignet sind diese s-Triazine zur Vernichtung von Flughafer in Getreidekulturen. Sie können einzeln oder in Mischungen verwendet werden, insbesondere in Mengen von je 0,125 bis 10 kg/ha. Ihre Applikationsform entsteht durch Zumischen von Hilfsstoffen, wie Träger-, Verdünnungs-, Lösungs-, Netz-, Haft-, Dispergier- und Emulgiermittel.

Sie können mit Herbiziden, Fungiziden, Insektiziden, Wachstumsregulatoren und Düngemitteln eingesetzt werden.



Croydon Printing Company Ltd.

## Verwendung von in 2-Stellung mit (substituierten) Aminogruppen substituierten 4-DL-Alkylester-α-alaninyl-6-chlor-s-Triazinen als Herbizide, insbesondere gegen Flughafer

### Stand der Technik

2-Chlor-4,6-bis-ethylamino-1,3,5-triazin (= Simazin vgl. CH-PS 329 277 und CH-PS 342 784 bzw. DE-AS 10 11 904) und 2-Chlor-4-ethylamino-6-isopropylamino-1,3,5-triazin (= Atrazin vgl. DE-AS 10 11 904) sind die Vertreter der Chlor-1,3,5-triazine, die seit der Mitte der 60er Jahre dieses Jahrhunderts zur Bekämpfung von Unkräutern und Schadgräsern bei verschiedenen Kulturpflanzen bisher die weiteste Verbreitung gefunden haben.

Als Nachteil der bisher bekannten Chlor-s-triazine stellte sich im allgemeinen ihre generelle Wirkung heraus, d. h. nicht nur die Schadgräser, sondern auch die Nutzpflanzen wurden durch sie geschädigt. Immerhin konnten bei einzelnen s-Triazin-Derivaten ganz spezifische Selektivitäten beobachtet werden, z. B. die der o. g. Verbindungen in Mais, Zuckerrohr oder Ananas. Bisher fehlen jedoch solche Herbizide aus dieser Stoffklasse, die in Winterweizen, Hafer, Gerste, Weizen, Raps, Reis, Zuckerrüben usw. selektiv wirken.

### Erfindungsbeschreibung

Überraschenderweise wurde gefunden, daß die Mischsubstitution mit einem grad- oder verzweigtkettigen oder cycloaliphatischen Amin und einem Alaninester zu Verbindungen mit einer sehr guten Selektivität in Getreidekulturen, wie Weizen, Gerste und Roggen, und mit gleichzeitig sehr guter Vernichtungswirkung gegen Unkräuter und Schadgräser führt.

Besonders bemerkenswert bei dieser Stoffgruppe ist die sehr gute Wirkung gegen Flughafer. Bekanntlich ist Flughafer wegen seiner botanischen Verwandtschaft zu Hafer, aber auch zu Gerste, Weizen und Roggen besonders schwierig in Getreidekulturen zu bekämpfen. Flughafer bildet an vielen Getreidestandorten ein erhebliches Problem, da Flughafer schneller als Nutzgetreide reift und seine Samen daher teilweise vor der Getreideernte bereits in den Boden gelangen. Außerdem ist es schwierig, bei der Saatgutreinigung Flughafersamen vom Getreidesaatgut zu trennen. S-Triazine mit guter Flughaferwirkung bei gleichzeitiger Selektivität in Getreidekulturen, wie Weizen, Gerste und Roggen, und besonders in Hafer sind bisher nicht bekannt.

Die erfindungsgemäßen Verbindungen, die den überraschenden Effekt der sehr guten Getreideselektivität bei gleichzeitig sehr guter Flughaferwirkung aufweisen, stellen daher eine wertvolle Bereicherung der herbiziden Mittel zur selektiven Unkrautbekämpfung dar.

Gegenüber anderen in der Literatur bekanntgewordenen Alaninderivaten wie Suffix (= 2(N-Benzoyl-3,4-dichlorphenylamino-)propionsäure-ethylester, vgl. DE-PS 16 43 527) weisen die erfindungsgemäßen Verbindungen den Vorteil der für Triazine bekannten Breitenwirkung gegen Unkräuter und andere Schadgräser (als Flughafer) auf.

Neben der sehr guten Getreideverträglichkeit zeichnen sich die erfindungsgemäßen Verbindungen durch ein breites Selektivitätsspektrum gegenüber zahlreichen anderen Kulturpflanzen aus. Die vorliegenden Verbindungen sind im Vorauflaufverfahren und/oder Nachauflaufverfahren u. a. auch selektiv in Sojabohnen, Baumwolle und Reis (lateinische Namen vergleiche Tabelle 2, Seite 14).

Der Einsatz der erfindungsgemäßen Verbindungen ist nicht nur auf Kulturen der o. g. Pflanzenarten beschränkt, er

kann auch erfolgreich in Kulturen anderer Pflanzen erfolgen.

In Abhängigkeit von der Konzentration können die Verbindungen auch zur Totalunkrautbekämpfung, z. B. auf Industrie- und Gleisanlagen oder auf Wegen und Plätzen mit und ohne Baumbewuchs, eingesetzt werden. Auch zur Unkrautbekämpfung in Dauerkulturen, wie z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen sind die erfindungsgemäßen Verbindungen geeignet.

Bei einigen Pflanzenarten wirken einige der erfindungsgemäßen Verbindungen hemmend auf das Längenwachstum, ohne jedoch zu Ertragsminderungen zu führen; sie können deshalb auch zusätzlich als wachstumsregulierende Mittel eingesetzt werden. Manche der neuen Verbindungen können auch als Defoliants, Desiccants, Krautabtötungsmittel und Keimhemmungsmittel verwendet werden.

Die Erfindung betrifft also die Verwendung von in 2-Stellung mit einer (substituierten) Aminogruppe substituierten 4-DL-Alkylester-α-alaninyl-6-chlor-s-triazinen der allgemeinen Formel

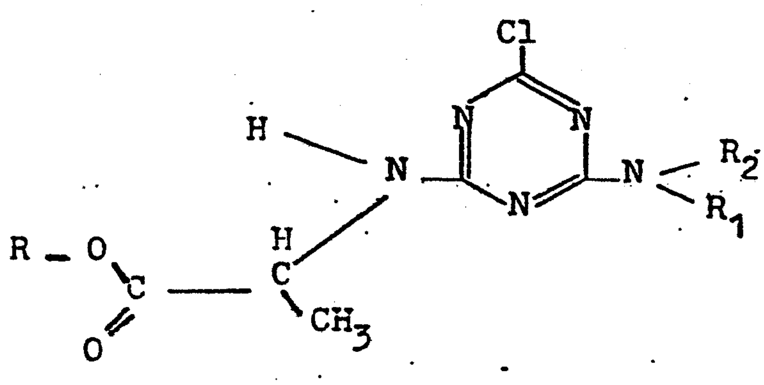 I,

in der $R_1$ einen gerad- oder verzweigtkettigen Alkyl-, Alkoxy-, Alkoxyalkyl oder Alkylol-Rest oder einen gegebenenfalls mit bis zu 5 Methylgruppen substituierter

Cyclopentyl- oder Cyclohexylring, $R_2$ einen Rest aus der Gruppe der Alkyle mit ein bis drei C-Atomen oder vorzugsweise ein H-Atom und R einen Rest aus der Gruppe der Alkyle mit ein bis drei C-Atomen bedeuten, in geeigneter Applikationsform zur selektiven Behandlung von Nutzpflanzenkulturen mit breitem Wirkungsspektrum bei Unkräutern und Schadgräsern vor und/oder nach dem Auflaufen der Saat.

<u>Herstellung der erfindungsgemäßen Racemate</u>

Die Herstellung erfolgt - unter Fortlassung der banalen Neutralisationsreaktionen des freigewordenen HCl nach den Schritten 1. und 2. mit NaOH nach folgendem Schema:

1.

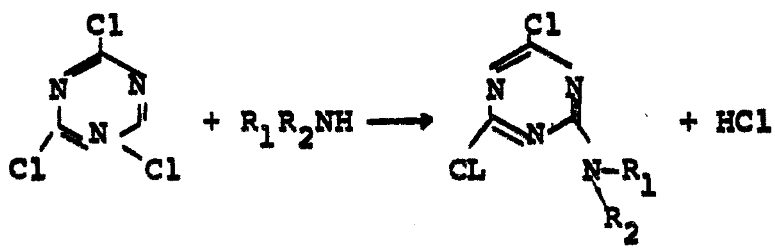

2.

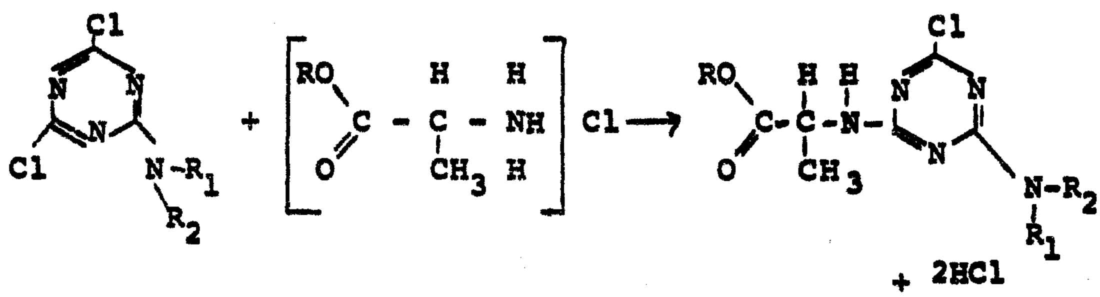

Die Symbole $R_1$, $R_2$ und R haben die auf den Seiten 3/4 angegebene Bedeutung. Die benutzten Lösungsmittel werden nachher durch Destillation abgetrennt und das entstandene Kochsalz durch Auswaschen bis zum Nachweis der Chloridfreiheit entfernt.
Die Ausbeute, bezogen auf Cyanurchlorid-Einsatz, beträgt 85 bis 92 %.

Da bei α-Alanin-Estern das C-Atom neben der COOR-Gruppe asymmetrisch ist, sind die Reaktionsprodukte dieser Verbindungen mit s-Triazinen stets Racemate.

Die Namen der hergestellten Racemate sind in Tabelle 1 a und die Substituenten $R_1$, $R_2$ und R und ihre Schmelzintervalle in Tabelle 1 b zusammengestellt. Dabei betreffen die Racemate 1 bis 18 jeweils den 4-DL-Ethylester-$\alpha$-alaninyl-6-chlor-s-triazin-Derivatenund die Racemate 101 bis 118 jeweils die entsprechenden 4-DL-Isopropylester-$\alpha$-alaninyl-6-chlor-s-triazin-Derivate.

Einzelheiten der Herstellung können auch aus den folgenden beiden Beispielen für je ein Ethylester- und ein Isopropylester-Derivat entnommen bzw. abgeleitet werden. Mit anderen Aminen ergeben sich jeweils nur geringfügige Verschiebungen der Reaktionstemperaturen und -zeiten, Ausbeuten und Aufarbeitungsverfahren.

O.Z. 3901

**Tabelle 1 a**

| Namen der hergestellten Racemate | Verbindung mit ... =ethyl = Nr. | Verbindung mit ... =isopropyl = Nr. |
|---|---|---|
| 2-Amino-4-DL-.....esteralaninyl-6-chlor-s-triazin | 1 | 101 |
| 2-Methylamino-4-DL-.....esteralaninyl-6-chlor-s-triazin | 2 | 102 |
| 2-Dimethylamino-4-DL-.....esteralaninyl-6-chlor-s-triazin | 3 | 103 |
| 2-Ethylamino-4-DL-.....esteraninyl-6-chlor-s-triazin | 4 | 104 |
| 2-Isopropylamino-4-DL-.....esteralaninyl-6-chlor-s-triazin | 5 | 105 |
| 2-n-Propylamino-4-DL-.....esteralaninyl-6-chlor-s-triazin | 6 | 106 |
| 2-tert.Butylamino-4-DL-.....esteralaninyl-6-chlor-s-triazin | 7 | 107 |
| 2-2'Hydroxy-ethylamino-4-DL-.....esteralaninyl-6-chlor-s-triazin | 8 | 108 |
| 2-2'Methoxy-ethylamino-4-DL-.....esteralaninyl-6-chlor-s-triazin | 9 | 109 |
| 2-3'Methoxy-propylamino-4-DL-.....esteralaninyl-6-chlor-s-triazin | 10 | 110 |
| 2-3'Ethoxy-propylamino-4-DL-.....esteralaninyl-6-chlor-s-triazin | 11 | 111 |
| 2-3'n-Butoxy-propylamino-4-DL-.....esteralaninyl-6-chlor-s-triazin | 12 | 112 |
| 2-3'n-Hexoxy-propylamino-4-DL-.....esteralaninyl-6-chlor-s-triazin | 13 | 113 |
| 2-3'isopropoxy-propylamino-4-DL-.....esteralaninyl-6-chlor-s-triazin | 14 | 114 |
| 2-Cyclopentylamino-4-DL-.....esteralaninyl-6-chlor-s-triazin | 15 | 115 |
| 2-Cyclohexylamino-4-DL-.....esteralaninyl-6-chlor-s-triazin | 16 | 116 |
| 2-TMCPamino-4-DL-.....esteralaninyl-6-chlor-s-triazin +) | 17 | 117 |
| 2-TMCamino-4-DL-.....esteralaninyl-6-chlor-s-triazin ++) | 18 | 118 |

0132512

Tabelle 1 a - Fortsetzung

+) TMCP bedeutet: 2,2,4- bzw. 2,4,4-Trimethylcyclopentyl-

++) TMC bedeutet: 3,3,5-Trimethylcyclohexanyl-

Tabelle 1 b

Liste der hergestellten 4-DL-Alkylester-α-alaninyl-6-chlor-s-triazin-Racemate mit verschiedenen Substituenten $R_1$, $R_2$ und R, R' und ihren Schmelzbereichen

| $R_1$ | $R_2$ | Verbindung mit $R = C_2H_5$ Nr. | Schmelzbereich °C | Verbindung mit $R = CH(CH_3)_2$ Nr. | Schmelzbereich °C |
|---|---|---|---|---|---|
| H | $-H$ | 1 | 112 - 117 | 101 | 146 - 151 |
| H | $-CH_3$ | 2 | 115 - 120 | 102 | 156 - 161 |
| $CH_3$ | $-CH_3$ | 3 | 120 - 125 | 103 | 158 - 163 |
| H | $-C_2H_5$ | 4 | 120 - 125 | 104 | 135 - 140 |
| H | $-CH(CH_3)_2$ | 5 | 101 - 106 | 105 | 103 - 108 |
| H | $-CH_2-CH_2-CH_3$ | 6 | 117 - 122 | 106 | 127 - 132 |
| H | $-C(CH_3)_3$ | 7 | 104 - 109 | 109 | 80 - 85 |
| H | $-CH_2-CH_2OH$ | 8 | 20 - 25 | 108 | 30 - 35 |
| H | $-CH_2-CH_2OCH_3$ | 9 | 20 - 25 | 109 | 120 - 125 |
| H | $-CH_2-CH_2-CH_2-OCH_3$ | 10 | 22 - 27 | 110 | 123 - 128 |
| H | $-CH_2-CH_2-CH_2OC_2H_5$ | 11 | 25 - 30 | 111 | 128 - 133 |
| H | $-CH_2-CH_2-CH_2OCH_2CH_2CH_2CH_3$ | 12 | 28 - 33 | 112 | 130 - 135 |
| H | $-CH_2-CH_2-CH_2OCH_2CH_2CH_2CH_2CH_2CH_3$ | 13 | 30 - 35 | 113 | 133 - 138 |
| H | $-CH_2-CH_2-CH_2-O-CH(CH_3)_2$ | 14 | 42 - 47 | 114 | 130 - 135 |

<u>Tabelle 1 b</u> - Fortsetzung

| $R_1$ | $R_2$ | Verbindung mit $R = C_2H_5$ Nr. | Schmelzbereich $^oC$ | Verbindung mit $R = CH(CH_3)_2$ Nr. | Schmelzbereich $^oC$ |
|---|---|---|---|---|---|
| H | 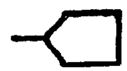 | 15 | 49 - 56 | 115 | 45 - 50 |
| H | 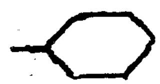 | 16 | 68 - 73 | 116 | 57 - 62 |
| H | 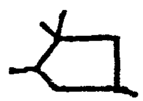 + 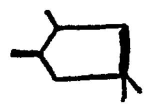 | 17 | 100 - 105 | 117 | 138 - 143 |
| H | 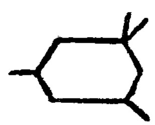 | 18 | 121 - 126 | 118 | 145 - 150 |

**Beispiel 1:** Racemat 2

Darstellung von 2-Methylamino-4-DL-ethylester-α-alaninyl-6-chlor-s-triazin

184,5 g (1,00 Mol) Cyanurchlorid werden in 900 ml Aceton gelöst und unter starkem Rühren in 1 100 ml Wasser bei 0 bis 2 °C in 30 min eingetropft. In die erhaltene Suspension werden dann in 30 min bei 0 bis 2 °C 31,0 g (1,00 Mol) Methylamin als 30 %ige wäßrige Lösung ebenfalls unter starkem Rühren eingetropft. Der Ansatz wird 30 min nachgerührt. Das Produkt bleibt dabei weitgehend ungelöst, der Ansatz liegt während der Reaktion mit Methylamin und Neutralisation der abgespaltenen HCl mit NaOH als gut rührbare Suspension vor.

Die zur Neutralisation benötigten 40,0 g (1,00 Mol) NaOH werden als 30 %ige Lösung in 60 min bei 0 bis 2 °C unter starkem Rühren eingetropft. Der pH-Wert 8,0 soll dabei nicht überschritten werden, er erreicht erst gegen Ende der Neutralisation den Wert 8,5 bis 9,0.

Zur weiteren Umsetzung werden 168,9 g (= 1,10 Mol) DL-Alanin-ethylester-hydrochlorid in Form einer 30 %igen ethanolischen Lösung bei -5 °C in 30 min unter starkem Rühren eingetropft.

Zur Neutralisation des Hydrochlorides werden anschließend 1,10 Mol NaOH als 30 %ige Lösung bei -5 °C in 60 min zugegeben. Die Suspension wird dann in 60 min unter Rühren auf 25 bis 30 °C erwärmt und bei dieser Temperatur mit weiteren 1,00 Mol einer 30 %igen NaOH versetzt. Die Neutralisation dauert 6 h, wobei der pH-Wert 8,0 nicht überschritten werden soll und erst gegen Ende den Wert 9,0 erreicht.

Zur Aufarbeitung werden Aceton und Alkohol aus dem Ansatz abdestilliert, das weiße kristalline Produkt abgesaugt und

mit Wasser chloridfrei gewaschen. Nach dem Trocknen im Vakuum bei 50 °C beträgt die Ausbeute, bezogen auf eingesetztes Cyanurchlorid, 89 %.

<u>Beispiel 2:</u> Racemat 115

Darstellung des 2-Cyclopentylamino-4-DL-isopropylester-α-alaninyl-6-chlor-s-triazin

184,5 g (1,00 Mol) Cyanurchlorid werden in 900 ml Aceton gelöst und unter starkem Rühren in 1 100 ml Wasser bei 0 bis 2 °C in 30 min eingetropft. In die erhaltene Suspension werden dann in 30 min bei 0 bis 2 °C 85,0 g (1,00 Mol) Cyclopentylamin ebenfalls unter starkem Rühren eingetropft. Der Ansatz wird 30 min nachgerührt, wobei das suspendierte Produkt größtenteils in Lösung geht.

Zur Neutralisation der abgespaltenen HCl werden anschließend 40,0 g (1,00 Mol) NaOH als 30 %ige Lösung zugetropft, Dauer 30 min. Der pH-Wert 8,0 soll dabei nicht überschritten werden, er darf erst gegen Ende der Neutralisation den Wert 8,5 bis 9,0 erreichen. Der Ansatz wird dabei stark gerührt und die Temperatur bei 0 bis 2 °C gehalten. Während der NaOH-Zugabe fällt das Produkt wieder feinverteilt aus und liegt dann als gut rührbare Suspension vor.

Nach Ende der NaOH-Zugabe werden 184,4 g (1,10 Mol) DL-Alanin-isopropylester-hydrochlorid als 30 %ige ethanolische Lösung bei -5 °C in 30 min unter starkem Rühren zugetropft.

Die Neutralisation des Hydrochlorides erfolgt dann durch Eintropfen von 146,7 g (1,10 Mol) 30 %ige NaOH bei der gleichen Temperatur unter starkem Rühren, Dauer 60 min. Der Ansatz enthält danach kein Festprodukt, ein Teil des Produktes scheidet sich als organische Schicht auf der wäßrigen Lösung ab. Anschließend wird im Laufe von 60 min

unter starkem Rühren auf 25 bis 30 °C erwärmt und bei dieser Temperatur mit weiteren 133,3 g (1,00 Mol) 30 %iger NaOH versetzt. Die Neutralisation dauert 6 h, wobei der pH-Wert 8,0 nicht überschritten werden soll und erst gegen Ende der pH-Wert 9,0 erreicht werden darf.

Zur Aufarbeitung werden Aceton und Ethanol aus dem Ansatz abdestilliert, wobei das Produkt als klebriges Harz aus der zurückbleibenden wäßrigen Lösung abgeschieden wird. Nach mehrmaligem Waschen mit Wasser bei 30 bis 40 °C und Trocknen im Vakuum bei 40 °C beträgt die Ausbeute 88 %, bezogen auf eingesetztes Cyanurchlorid.

Applikation der Wirkstoffe

Die Wirkstoffe der erfindungsgemäßen Verbindungen können je nach Kultur in einer Menge von 0,125 bis 10 kg/ha, vorzugsweise von 0,5 bis 2,5 kg/ha, appliziert werden. Es können sowohl Racemate entsprechend einer Formel (Verbindung mit einem Wert für $R_1$, $R_2$ und R) als auch Gemische von erfindungsgemäßen Verbindungen (d. h. solche mit verschiedenen Bedeutungen für $R_1$ und/oder $R_2$ und/oder R) zur Bekämpfung von Unkräutern und Ungräsern eingesetzt werden.

Außerdem ist es üblich, bei der Applikation ein oder mehrere Hilfsmittel aus der Gruppe der Träger-, Verdünnungs-, Lösungs-, Netz-, Haft-, Dispergier- und Emulgiermittel den Wirkstoffen zuzusetzen.

Auf die Zumischung anderer Herbizide bzw. der gleichzeitige Einsatz von Fungiziden, Insektiziden, Wachstumsregulatoren usw. mit den erfindungsgemäßen Wirkstoffen bzw. Wirkstoffgemischen ist möglich.

Schließlich können die erfindungsgemäßen Wirkstoffe oder Wirkstoffgemische, ggf. mit einem oder mehreren Vertretern

aus den o. g. Stoffgruppen, Mineral- und Kunstdüngern oder Bodenverbesserungsmitteln zugesetzt und mit ihnen zusammen verteilt werden.

Im Vorauflauf erfolgt die Applikation auf die Oberfläche vor dem Keimen der Samen und im Nachauflauf auf die Blattoberfläche im zweiten bis dritten Blattstadium.

Gewächshaus-Versuchsreihe mit 4, 3 und 2 kg/ha

Für die meisten der erfindungsgemäßen Verbindungen wurden Gewächshaus-Versuche mit einer Aufwandmenge von 4, 3 und 2 kg/ha, bezogen auf reinen Wirkstoff, an den in der Tabelle 2 (mit ihrer deutschen und lateinischen Bezeichnungen) zusammengestellten Testpflanzen durchgeführt.

Die jeweils benötigten Wirkstoffmengen wurden dabei in 1 000 l Wasser/ha suspendiert. Jeweils 3 Wochen nach der Behandlung der Kulturen mit dem Wirkstoff wurde bonitiert. Die Auswertung der Versuchsergebnisse erfolgte durch die übliche Benotung, bei der

1 = volle Wirkung = Abtötung der Pflanzen und
5 = keine Wirkung = Pflanzen wie unbehandelt

bedeuten.

In den Tabellen 3 und 4 wurden nur die Ergebnisse für die Racemate Nr. 4, 104 und 105 sowie von 1, bzw. 2 Vergleichssubstanzen aufgeführt, und zwar ist

Vergleichsmittel 1 = Atrazin = 2-Ethylamino-4-isopropylamino-6-chlor-1,3,5-triazin und

Vergleichsmittel 2 = Suffix = 2(N-Benzoyl-3,4-dichlorphenylamino)-propionsäure-ethylester

**Tabelle 2:** Botanische Namen der Testpflanzen

| deutsche Bezeichnung | lateinische Bezeichnung |
|---|---|
| Ackerfuchsschwanz | Alopecurus myosuroides |
| Baumwolle | Gossypium herbaceum |
| Flughafer | Avena fatua |
| Gerste | Hordeum vulgare |
| Hafer | Avena sativa |
| Kamille | Matricaria maritima |
| Mais | Zea mays |
| Sojabohnen | Glycine max |
| Taubnessel | Lamium purpureum |
| Tomate | Lycopersicum esculentum |
| Vogelmiere | Stellaria media |
| Weißer Gänsefuß | Chenopodium album |
| Weizen | Triticum aestivum |
| Windhalm | Apera spica-venti |
| Winterraps | Brassica napus |

<u>Tabelle 3</u>

**Herbizide Wirkung der erfindungsgemäßen Verbindungen in den Aufwandmengen von 4, 3 und 2 kg Wirkstoff/ha. Applikation <u>vor</u> dem Auflaufen der Pflanzen.**

| Testpflanzen | Beispiel 4 | | | Beispiel 5 | | | Beispiel 104 | | | Beispiel 105 | | |
|---|---|---|---|---|---|---|---|---|---|---|---|---|
| | 4 | 3 | 2 | 4 | 3 | 2 | 4 | 3 | 2 | 4 | 3 | 2 |
| Winterraps | 4 | 4 | 4 | 5 | 5 | 5 | 4 | 4 | 4 | 4 | 4 | 4 |
| Tomate | 1 | 3 | 3 | 5 | 5 | 5 | 1 | 1 | 1 | 3 | 3 | 4 |
| Hafer | 3 | 3 | 5 | 5 | 5 | 5 | 3 | 3 | 4 | 3 | 3 | 4 |
| Flughafer | 1 | 2 | 2 | 3 | 4 | 4 | 1 | 3 | 3 | 4 | 4 | 4 |
| Ackerfuchsschwanz | 1 | 1 | 1 | 3 | 3 | 5 | 1 | 1 | 1 | 1 | 1 | 2 |
| Windhalm | 1 | 1 | 1 | 3 | 3 | 3 | 1 | 1 | 1 | 1 | 1 | 1 |
| Taubnessel | 1 | 1 | 1 | 3 | 3 | 4 | 1 | 1 | 1 | 1 | 1 | 1 |
| Vogelmiere | 1 | 1 | 1 | 5 | 5 | 5 | 1 | 1 | 1 | 1 | 1 | 1 |
| Kamille | 1 | 1 | 1 | 4 | 5 | 5 | 1 | 1 | 1 | 1 | 1 | 1 |
| Weißer Gänsefuß | 1 | 1 | 1 | 4 | 5 | 5 | 1 | 1 | 1 | 1 | 1 | 1 |
| Gerste | 5 | 5 | 5 | 5 | 5 | 5 | 5 | 5 | 5 | 5 | 5 | 5 |
| Weizen | 5 | 5 | 5 | 5 | 5 | 5 | 5 | 5 | 5 | 5 | 5 | 5 |
| Sojabohnen | 5 | 5 | 5 | 5 | 5 | 5 | 5 | 5 | 5 | 5 | 5 | 5 |
| Baumwolle | 5 | 5 | 5 | 5 | 5 | 5 | 5 | 5 | 5 | 5 | 5 | 5 |
| Mais | 5 | 5 | 5 | 5 | 5 | 5 | 5 | 5 | 5 | 5 | 5 | 5 |

**Tabelle 3 - Fortsetzung**

| Testpflanzen | Vergleichsmittel 1 | | |
|---|---|---|---|
| | 4 | 3 | 2 |
| Winterraps | 1 | 1 | 1 |
| Tomate | 1 | 1 | 1 |
| Hafer | 1 | 1 | 1 |
| Flughafer | 1 | 1 | 1 |
| Ackerfuchsschwanz | 1 | 1 | 1 |
| Windhalm | 1 | 1 | 1 |
| Taubnessel | 1 | 1 | 1 |
| Vogelmiere | 1 | 1 | 1 |
| Kamille | 1 | 1 | 1 |
| Weißer Gänsefuß | 1 | 1 | 1 |
| Gerste | 1 | 1 | 1 |
| Weizen | 1 | 1 | 1 |
| Sojabohnen | 1 | 1 | 1 |
| Baumwolle | 2 | 2 | 3 |
| Mais | 5 | 5 | 5 |

Tabelle 4

Herbizide Wirkung der erfindungsgemäßen Verbindungen in den Aufwandmengen von 4, 3 und 2 kg Wirkstoff/ha. Applikation nach dem Auflaufen der Pflanzen.

| Testpflanzen | Beispiel 4 | | | Beispiel 5 | | | Beispiel 104 | | | Beispiel 105 | | |
|---|---|---|---|---|---|---|---|---|---|---|---|---|
| | 4 | 3 | 2 | 4 | 3 | 2 | 4 | 3 | 2 | 4 | 3 | 2 |
| Winterraps | 1 | 1 | 3 | 1 | 1 | 1 | 1 | 1 | 1 | 1 | 1 | 4 |
| Tomate | 1 | 1 | 3 | 5 | 5 | 5 | 1 | 4 | 4 | 3 | 3 | 4 |
| Hafer | 3 | 3 | 4 | 3 | 3 | 4 | 3 | 3 | 4 | 3 | 3 | 3 |
| Flughafer | 1 | 1 | 1 | 1 | 1 | 1 | 1 | 1 | 1 | 1 | 1 | 1 |
| Ackerfuchsschwanz | 1 | 1 | 1 | 1 | 1 | 1 | 1 | 1 | 1 | 1 | 1 | 1 |
| Windhalm | 1 | 1 | 1 | 1 | 1 | 1 | 1 | 1 | 1 | 1 | 1 | 1 |
| Taubnessel | 1 | 1 | 1 | 1 | 1 | 1 | 1 | 1 | 1 | 1 | 1 | 1 |
| Vogelmiere | 1 | 1 | 1 | 1 | 1 | 1 | 1 | 1 | 1 | 1 | 1 | 1 |
| Kamille | 1 | 1 | 1 | 1 | 2 | 3 | 1 | 1 | 1 | 1 | 1 | 1 |
| Weißer Gänsefuß | 1 | 1 | 1 | 1 | 3 | 3 | 1 | 1 | 1 | 1 | 1 | 1 |
| Gerste | 4 | 4 | 5 | 4 | 4 | 5 | 3 | 4 | 5 | 3 | 4 | 4 |
| Weizen | 4 | 4 | 5 | 3 | 4 | 5 | 4 | 4 | 5 | 3 | 4 | 4 |
| Sojabohnen | 5 | 5 | 5 | 5 | 5 | 5 | 5 | 5 | 5 | 5 | 5 | 5 |
| Baumwolle | 5 | 5 | 5 | 5 | 5 | 5 | 5 | 5 | 5 | 5 | 5 | 5 |
| Mais | 5 | 5 | 5 | 5 | 5 | 5 | 5 | 5 | 5 | 5 | 5 | 5 |

O.Z. 3901

**Tabelle 4 - Fortsetzung**

| Testpflanzen | Vergleichsmittel 1 | | | Vergleichsmittel 2 | | |
|---|---|---|---|---|---|---|
| | 4 | 3 | 2 | 4 | 3 | 2 |
| Winterraps | 1 | 1 | 1 | 5 | 5 | 5 |
| Tomate | 1 | 1 | 1 | 5 | 5 | 5 |
| Hafer | 1 | 1 | 1 | 3 | 3 | 3 |
| Flughafer | 1 | 1 | 1 | 1 | 1 | 1 |
| Ackerfuchsschwanz | 1 | 1 | 1 | 1 | 1 | 1 |
| Windhalm | 1 | 1 | 1 | 1 | 1 | 2 |
| Taubnessel | 1 | 1 | 1 | 5 | 5 | 5 |
| Vogelmiere | 1 | 1 | 1 | 5 | 5 | 5 |
| Kamille | 1 | 1 | 1 | 5 | 5 | 5 |
| Weißer Gänsefuß | 1 | 1 | 1 | 3 | 5 | 5 |
| Gerste | 1 | 1 | 1 | 4 | 5 | 5 |
| Weizen | 1 | 1 | 1 | 5 | 5 | 5 |
| Sojabohnen | 1 | 1 | 1 | 5 | 5 | 5 |
| Baumwolle | 1 | 1 | 1 | 5 | 5 | 5 |
| Mais | 5 | 5 | 5 | 5 | 5 | 5 |

Die in den Tabellen 3 und 4 zusammengestellten Ergebnisse belegen, daß die erfindungsgemäßen Verbindungen sowohl im Vor- als auch im Nachauflauf eine hervorragende Wirkung gegenüber zahlreichen Unkräutern und Schadgräsern, wie Flughafer, Ackerfuchsschwanz, Windhalm, Taubnessel, Vogelmiere, Kamille und Weißer Gänsefuß, aufweisen. Dabei ist die Selektivität in verschiedenen Kulturen, wie Gerste, Weizen, Hafer, Soja, Baumwolle, erheblich besser als bei dem Vergleichsmittel 1 (Atrazin). Gegenüber dem Vergleichsmittel 2 (Suffix), das dem Stand der Technik nach als Flughaferherbizid gilt, besteht ein wesentlich erweitertes Wirkungsspektrum gegenüber zweikeimblättrigen Unkräutern.

In Haferkulturen sollten die erfindungsgemäßen Racemate nur in Mengen unter 2 kg/ha eingesetzt werden, da sonst auch gewisse Schädigungen bei den Nutzpflanzen beobachtet werden müssen.

Gewächshaus-Versuchsreihen mit Aufwandmengen von 1, 0,5, 0,25 und 0,125 kg/ha im Nachauflauf

Wegen der vorhandenen hohen herbiziden Wirkung der erfindungsgemäßen Verbindungen wurden zusätzlich noch niedrigere Aufwandmengen (1, 0,5, 0,25 und 0,125 g Wirkstoff/ha) im Nachauflauf geprüft.

Nach Tabelle 5 ist die Wirkung der erfindungsgemäßen Verbindungen gegen Flughafer dem Vergleichsmittel 2 ebenbürtig. Das Wirkungsspektrum der erfindungsgemäßen Verbindungen ist jedoch gegenüber diesem Vergleichsmittel deutlich größer. Im Vergleich zu s-Triazinen wie dem Vergleichsmittel 1 ist die Kulturselektivität in Getreidearten (Gerste, Weizen) auch im Nachauflauf gegeben, so daß im Gegensatz zu dem Vergleichsmittel 1 die erfindungsgemäßen Verbindungen zur Flughafer- und Ackerfuchsschwanzbekämpfung in Getreide eingesetzt werden können.

O.Z. 3901

**Tabelle 5**

Herbizide Wirkung der erfindungsgemäßen Verbindungen in den Aufwandmengen von 1, 0,5, 0,25 und 0,125 kg/ha. Applikation nach dem Auflaufen der Pflanzen.

| Testpflanzen | Beispiel 4 | | | | Beispiel 104 | | | | Beispiel 105 | | | |
|---|---|---|---|---|---|---|---|---|---|---|---|---|
| | 1 | 0,5 | 0,25 | 0,125 | 1 | 0,5 | 0,25 | 0,125 | 1 | 0,5 | 0,25 | 0,125 |
| Flughafer | 1 | 1 | 1 | 1 | 1 | 1 | 1 | 2 | 1 | 1 | 1 | 2 |
| Ackerfuchsschwanz | 1 | 1 | 1 | 2 | 1 | 1 | 1 | 2 | 1 | 1 | 2 | 2 |
| Taubnessel | 1 | 1 | 1 | 2 | 1 | 1 | 1 | 2 | 1 | 1 | 1 | 2 |
| Vogelmiere | 1 | 1 | 1 | 3 | 1 | 1 | 1 | 3 | 1 | 1 | 2 | 3 |
| Kamille | 1 | 1 | 1 | 1 | 1 | 1 | 1 | 1 | 1 | 1 | 1 | 1 |
| Weißer Gänsefuß | 1 | 1 | 1 | 1 | 1 | 1 | 1 | 1 | 1 | 1 | 1 | 1 |
| Gerste | 5 | 5 | 5 | 5 | 5 | 5 | 5 | 5 | 5 | 5 | 5 | 5 |
| Weizen | 5 | 5 | 5 | 5 | 5 | 5 | 5 | 5 | 5 | 5 | 5 | 5 |
| Roggen | 5 | 5 | 5 | 5 | 5 | 5 | 5 | 5 | 5 | 5 | 5 | 5 |

**Tabelle 5 - Fortsetzung**

| Testpflanzen | Vergleichsmittel 1 | | | | Vergleichsmittel 2 | | | |
|---|---|---|---|---|---|---|---|---|
| | 1 | 0,5 | 0,25 | 0,125 | 1 | 0,5 | 0,25 | 0,125 |
| Flughafer | 1 | 1 | 1 | 1 | 1 | 1 | 1 | 2 |
| Ackerfuchsschwanz | 1 | 1 | 1 | 2 | 1 | 1 | 1 | 2 |
| Taubnessel | 1 | 1 | 1 | 2 | 5 | 5 | 5 | 5 |
| Vogelmiere | 1 | 1 | 2 | 3 | 5 | 5 | 5 | 5 |
| Kamille | 1 | 1 | 1 | 1 | 5 | 5 | 5 | 5 |
| Weißer Gänsefuß | 1 | 1 | 1 | 1 | 5 | 5 | 5 | 5 |
| Gerste | 1 | 1 | 1 | 3 | 5 | 5 | 5 | 5 |
| Weizen | 1 | 1 | 1 | 2 | 5 | 5 | 5 | 5 |
| Roggen | 1 | 1 | 1 | 1 | 5 | 5 | 5 | 5 |

Patentansprüche:

1. Verwendung von in 2-Stellung mit einer (substituierten) Aminogruppe substituierten 4-DL-Alkylester-α-alaninyl-6-chlor-s-triazinen der allgemeinen Formel

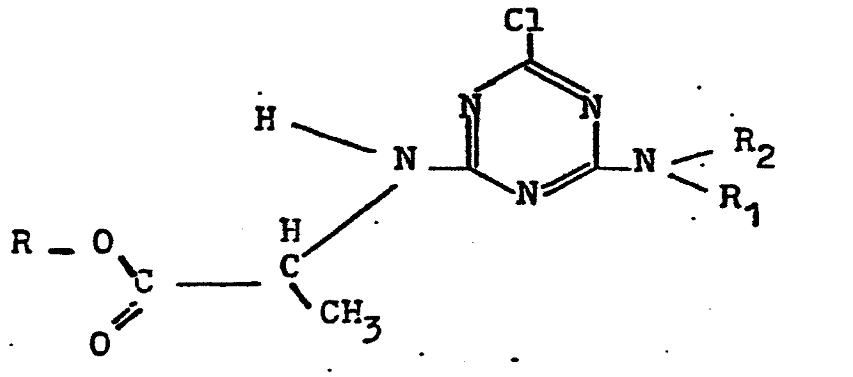

I,

in der $R_1$ einen gerad- oder verzweigtkettigen Alkyl-, Alkoxy-, Alkoxyalkyl oder Alkylol-Rest oder einen gegebenenfalls mit bis zu 5 Methylgruppen substituierter Cyclopentyl- oder Cyclohexylring, $R_2$ einen Rest aus der Gruppe der Alkyle mit ein bis drei C-Atomen oder vorzugsweise ein H-Atom und R einen Rest aus der Gruppe der Alkyle mit ein bis drei C-Atomen bedeuten, in geeigneter Applikationsform zur selektiven Behandlung von Nutzpflanzenkulturen mit breitem Wirkungsspréktrum bei Unkräutern und Schadgräsern vor und/oder nach dem Auflaufen der Saat.

2. Verwendung von s-Triazinen der Formel I zur Vernichtung von Flughafer in Getreidekulturen.

3. Verwendung von Mischungen von zwei oder mehreren Wirkstoffen gemäß Formel I, bei denen sich die Einzelwirkstoffe durch unterschiedliche Substituenten $R_1$ und/oder $R_2$ und/oder R voneinander unterscheiden, nach Anspruch 1 und/oder 2.

4. Verwendung eines oder mehrerer der Wirkstoffe nach Anspruch 1 und/oder 2 und/oder 3 in Mengen von je 0,125 bis 10 kg/ha, vorzugsweise von 0,5 bis 2,5 kg/ha.

5. Verwendung eines oder mehrerer der Wirkstoffe nach Anspruch 1 und/oder 2 und/oder 3 und/oder 4,
dadurch gekennzeichnet,
daß man die jeweilige Applikationsform durch Zumischen von Hilfsmitteln aus der Gruppe der Träger-, Verdünnungs-, Lösungs-, Netz-, Haft-, Dispergier- und/oder Emulgiermittel gewinnt.

6. Verwendung eines oder mehrerer der Wirkstoffe nach Anspruch 1 und einem oder mehreren der Ansprüche 2 bis 5,
gekennzeichnet durch
die Zumischung von Stoffen aus der Gruppe der Herbizide, Fungizide, Insektizide, Wachstumsregulatoren usw.

7. Verwendung eines oder mehrerer der Wirkstoffe nach Anspruch 1 und gegebenenfalls nach einem oder mehreren der Ansprüche 2 bis 6 als Zusatz zu Düngemitteln.

Europäisches Patentamt

# EUROPÄISCHER RECHERCHENBERICHT

0132512

Nummer der Anmeldung

EP 84 10 4671

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | | | A 01 N 43/70 |
| A | US-A-4 229 206 (S.R. SIEMER)<br>--- | 1-7 | |
| A | GB-A-1 153 245 (DEGUSSA)<br>----- | 1-7 | |
| | | | RECHERCHIERTE SACHGEBIETE (Int. Cl. ³) |
| | | | A 01 N |

Der vorliegende Recherchenbericht wurde für alle Patentansprüche erstellt.

| Recherchenort | Abschlußdatum der Recherche | Prüfer |
|---|---|---|
| DEN HAAG | 10-10-1984 | DECORTE D. |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503. 03.82